(19) Europäisches Patentamt
European Patent Office
Office européen des brevets

(11) Publication number: **0 043 612**
**B1**

(12) # EUROPEAN PATENT SPECIFICATION

(45) Date of publication of patent specification: 25.07.84

(21) Application number: 81200686.4

(22) Date of filing: 17.06.81

(51) Int. Cl.³: C 08 G 59/08, C 08 G 65/28, C 08 L 63/02, C 07 D 303/27, C 09 D 3/58, C 09 D 5/40

(54) **Polyglycidyl ethers of diphenylol alkanes, preparation and use in curable compositions.**

(30) Priority: 08.07.80 GB 8022299

(43) Date of publication of application:
13.01.82 Bulletin 82/2

(45) Publication of the grant of the patent:
25.07.84 Bulletin 84/30

(84) Designated Contracting States:
CH DE FR GB IT LI NL

(56) References cited:
CH - A - 544 801
DE - A - 1 645 191
DE - A - 2 828 420
DE - A - 2 943 879
FR - A - 1 066 034
FR - A - 2 027 809
GB - A - 666 732
GB - A - 752 226
GB - A - 959 286

The file contains technical information submitted after the application was filed and not included in this specification

(73) Proprietor: SHELL INTERNATIONALE RESEARCH MAATSCHAPPIJ B.V.
Carel van Bylandtlaan 30
NL-2596 HR Den Haag (NL)

(72) Inventor: Ramsbotham, John
Badhuisweg 3
NL-1031 CM Amsterdam (NL)
Inventor: van Gogh, Johan
Badhuisweg 3
NL-1031 CM Amsterdam (NL)

(74) Representative: Puister, Antonius Tonnis, Mr. et al.
P.O. Box 302
NL-2501 CH The Hague (NL)

EP 0 043 612 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to novel polyglycidyl ethers of diphenyl alkanes, to their preparation, and to their use, for example in curable compositions, or for the preparation of water-soluble or water-dispersible paint binders.

Polyglycidyl ethers of 2,2-bis(4-hydroxyphenyl)propane (abbreviated DPP) of large variety in molecular weight are well-known commercial products. They are also known as epoxy resins, and find use on a large scale as main resinous component in curable or thermosetting compositions.

For particular reference is made to the books "Handbook of Epoxy Resins" by Henry Lee and Kris Neville, dated 1967, and "Uses of Epoxy Resins" by W. G. Potter, dated 1975.

These epoxy resins are compounds having the general formula:

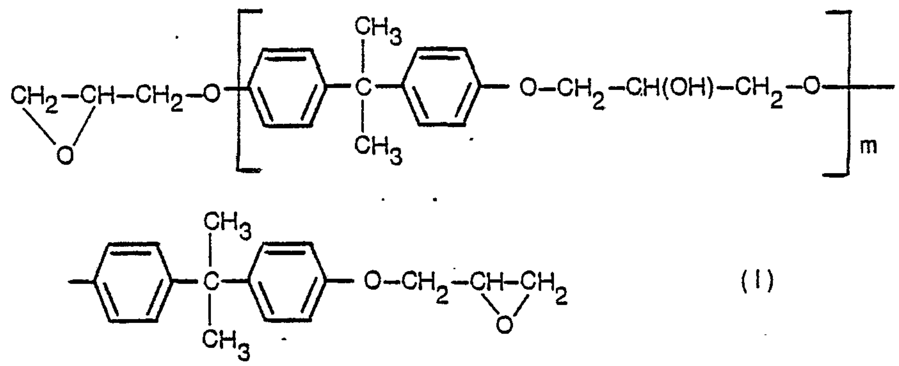

(I)

wherein m is a number having an average value from 0 to 12. Part of the glycidyl groups in the above formula may have been replaced by glycol groups, due to the methods of preparation. Compounds of the above formula wherein m is 0—1 are liquid or semi-liquid at room temperature, compounds wherein m is at least 2 are solids at room temperature.

In French Patent Specification No. 1,066,034 there are disclosed various polyglycidyl ethers of polyphenylol alkanes and general methods for their preparation. These polyglycidyl ethers contain at least three glycidyl moieties per molecule and are derived from phenol-aldehyde condensation products containing at least three phenol moieties per molecule. Aldehydes which may be condensed with the phenol include formaldehyde, acetaldehyde, propionaldehyde, butyraldehyde, heptaldehyde, benzaldehyde, furfuraldehyde and glyoxal.

For some purposes, however, some properties of epoxy resin from DPP need improvement. For example, the viscosity of the liquid resins is rather high, usually 10 Pa.s (25°C) or more; when a resin of lower viscosity is required, the DPP-based resin has to be diluted with a small amount of a reactive diluent: a mono-epoxide or di-epoxide of low viscosity and other structure, usually a glycidyl ether of a monohydric alcohol or a glycol. These additives, however, will usually reduce the resistance of the cured products to heat, solvents, and/or chemicals.

Further, the glycidyl ethers of DPP have low compatibility for aliphatic hydrocarbons (which are attractive as cheap solvents), and have to be dissolved or diluted with the more expensive polar solvents, such as ketones, glycol ethers or esters.

For some purposes, a better flexibility of cured products is required. This can be obtained by use of flexibilizers (non-reactive), flexibilizing epoxides, and/or flexibilizing curing agents. Apart from price considerations (the additives are usually more expensive) there is usually a reduction in other useful properties, such as resistance to heat, solvents, and/or chemicals.

Summarising, there is still a need for epoxy resins of the bisphenol type, having good compatibility with aliphatic hydrocarbon solvents, and providing with usual curing agents cured products of improved flexibility, without undue reduction in adhesion, hardness, and resistance to heat and chemicals, whereas the liquid resins of that type should have a low viscosity.

Further, epoxy resins of the bisphenol type are often used for preparation of aqueous thermosetting binders for paints, in particular for application by electrodeposition techniques. Here a further improvement in flow at electrodeposition or cure would be desirable, preferably at simplified preparation techniques.

The invention provides novel epoxy resins meeting these requirements. The novel epoxy resins are defined as polyglycidyl ethers of diphenylol alkanes having the general formula

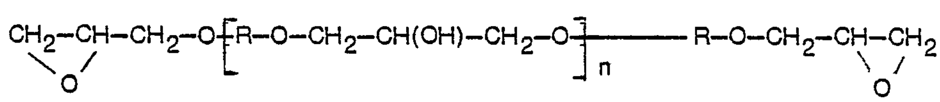

(II)

wherein n is a number of from 0 to 12 and R is the hydrocarbon residue of a diphenylol alkane,

characterized in that from 1 to (n + 1) groups R in the above molecule have the formula

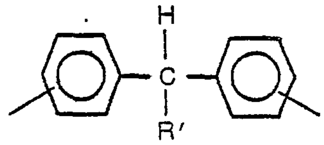

(III)

wherein R′ is a n-hexyl group and wherein the weight ratio of para, para′-isomer to ortho, para′-isomer is from 90:10 to 40:60.

In this general formula a part of the terminal epoxy groups may be replaced by glycol groups, due to the methods of preparation. Polyglycidyl therefore means that the compounds contain on average more than one epoxy group per molecule.

Novel epoxy resins according to the invention can be prepared, for example, by reaction of a diphenylol alkane with epichlorohydrin or with a polyepoxide, wherein the diphenylol alkane has the general formula

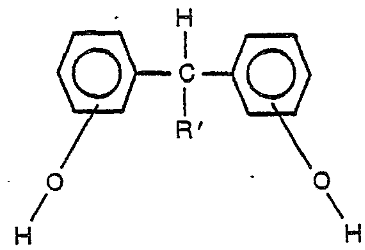

wherein R′ is a n-hexyl group and the weight ratio para, para′-isomer to ortho, para′-isomer is from 90:10 to 40:60.

In formula II, n is preferably a number of from 0 to 4, and the weight ratio of p, p′ to o,p′-isomer from 80:20 to 40:60.

Preferred are the "liquid" polyglycidyl ethers derived from 1,1 bis(hydroxyphenyl)n-heptane isomer mixtures wherein n has an average value of from 0 to 1. These liquids have a low viscosity, they have no tendency to crystallize, and can be easily diluted with aliphatic hydrocarbon solvents. These liquid epoxy resins can be prepared in high yields by reaction of the corresponding mixture of bisphenol isomers with epichlorohydrin, or dichlorohydrin, and an alkali metal hydroxide, if desired in the presence of catalysts or solvents.

The mixture of bisphenol isomers can be prepared in high yield by reaction of n-heptanal, with a molar excess of phenol in the presence of hydrochloric acid, if desired in the presence of mercapto cocatalysts, at moderate temperatures; the excess of phenol and the catalyst can be removed after the reaction, for example by distillation. The product, the residue, is a mixture of bisphenol isomers, containing mainly p,p′-isomer and o,p′-isomer (in a weight ratio of from 80:20 to 40:60), and may contain up to 10% by weight of higher condensates (trihydric phenols). The weight ratio p,p′-isomer to o,p′-isomer is usually from 65:35 to 55:45, and the amount of o,o′-isomer can normally be neglected.

At room temperature and slightly above, these mixtures of bisphenol isomers are liquid to semisolid, and can be diluted easily with solvent or reagent required for conversion into glycidyl ethers. For the latter reaction there is no need to separate the isomers, or to enrich the content of one of the isomers, although that may be envisaged.

The mixture of bisphenol isomers can, for example, be diluted easily with epichlorohydrin and/or alcohols, such as methanol, ethanol, n-propanol, isopropanol, n-butanol, iso-butanol, and sec.-butanol.

The mixture of bisphenol isomers can be converted into diglycidyl ethers by reaction with epichlorohydrin and alkali metal hydroxide, by the methods known for the preparation of diglycidyl ethers of 2,2-bis(4-hydroxyphenyl)propane.

The liquid new epoxy resins can be prepared in general by reacting the bisphenol with a molar excess of epichlorohydrin, if desired first in a pre-reaction in the presence of a catalyst which promotes formation of chlorohydrin ethers, whereafter the equivalent amount, or preferably an excess, of alkali metal hydroxide is added in one or more stages for dehydrohalogenation.

So-called solid polyglycidyl ethers (wherein n in the above formula (II) is at least 2) can be prepared by reacting the mixture of bisphenol isomers in aqueous medium with less epichlorohydrin and with alkali metal hydroxide in one stage.

Polyglycidyl ethers of formula II wherein n is at least 2 can also be prepared by reaction of an underdose of a bisphenol with a diglycidyl ether of a bisphenol in the presence of a small amount of a catalyst, for example a tertiary amine, a quaternary ammonium salt, or a quaternary phosphonium salt, for example as described in the British patents 1,204,760 and 1,364,804. Various types of polyglycidyl ethers according to the invention can be prepared by this method: a bisphenol of formula IV can be

reacted with a diglycidyl ether of the same bisphenol, or with a diglycidyl ether of 2,2-bis(4-hydroxyphenyl) propane, or further the latter bisphenol can be reacted with a diglycidyl ether of bisphenol of formula IV. The resulting polyglycidyl ether will then contain from 1 to n + 1 bisphenol residues of formula III per molecule, the remaining bisphenol residues being derived from the diphenylol propane.

Polyglycidyl ethers according to the invention can be modified by reaction with all types of modifying agents known for the usual epoxy resins, for example with fatty acid to prepare epoxy resin esters, with amines (excess) to prepare soluble epoxy resin/amine adducts, with dicarboxylic acids (underdose) to prepare epoxy resins having a dicarboxylic acid ester group in the chain.

The polyglycidyl ethers according to the invention can be used in particular as base materials for the preparation of anionic or cationic aqueous thermosetting binders for paints, for application by electrodeposition. Some of such methods have been described in the British patents 1409728 and 1556201, and in the European patent applications 78200031 and 79200700.

Polyglycidyl ethers according to the invention can be converted to hard resinous materials by mixing and reacting with an epoxy resin curing agent. Useful curing agents are amino compounds, in particular those having at least two amino hydrogen atoms per molecule. Examples are aliphatic polyamines, aromatic polyamines, soluble adducts thereof with mono-epoxides and/or di-epoxides, amino amides derived from aliphatic polyamines and carboxylic acids, heterocyclic polyamines. The cure with amino compounds may be accelerated by phenols or monocarboxylic acids. Other useful curing agents are polycarboxylic acids and polycarboxylic acid anhydrides; the cure may be accelerated by small amounts of catalysts, such as tertiary amines, for example benzyldimethylamine, or 2,4,6-tris(dimethylaminomethyl)phenol, stannous salts, and others. Further curing agents are polyisocyanates, phenolic resins, and aminoplast resins.

Solvents, diluents, extenders (coal tar, asphaltic bitumen), fillers, and pigments may also be added, depending on the intended use. Polyglycidyl ethers according to the invention wherein n has a value from 0 to 1 can be used as main binder component in coating compositions, in particular for solventless or high-solids coating compositions, or for use in civil engineering, for example for coverage of floors, road surfaces, bridge decks, as adhesive, or for casting, moulding, laminating, for example for electrical applications. Further uses to be mentioned are as a binder in grouts, as resinous component in tooling operations, and for cable jointing compositions. In flooring compositions these polyglycidyl ethers allow a very high filler loading.

Polyglycidyl ethers according to the invention wherein n is more than 1 may find a use for coating, laminating, or as adhesive.

The use of polyglycidyl ethers according to the invention provides in general an improved solubility in aliphatic hydrocarbon solvents and very good pigment wetting, and after cure an excellent adhesion on the usual substrates, improved flexibility, and very good resistance to chemicals.

Polyglycidyl ethers according to the invention may for many purposes be mixed with other polyepoxides, for example with the conventional diglycidyl ethers of 2,2-bis(4-hydroxyphenyl)propane.

The invention is illustrated by examples.

Example I

Preparation of diphenylol heptane

Phenol (2820 g; 30 moles) is melted, and saturated with gaseous HCl at 50°C with vigorous stirring during 15 minutes (HCl content: 1%w). n-heptanal (228 g; 2 moles) is added in 15 minutes under a slight $N_2$-stream. The temperature rose from 50°C to 60°C. The mixture was stirred during a further 15 minutes at 50—60°C.

Water, HCl and the excess of phenol were removed by vacuum distillation: first at 2000 Pa at up to 80°C where the bulk of phenol comes over, followed by vacuum steam distillation (10000 Pa, bottom temperature 120°C), and finally under $N_2$ (5300 Pa, bottom temperature 140°C).

The residue (535 g), crude 1,1-diphenylol heptane (DPH), contained (HPL chromatography):

| | |
|---|---|
| para,para′-isomer | 56%w |
| ortho,para′-isomer | 37%w |
| ortho,ortho′-isomer | negligible |
| polyphenols (trimers) | 6%w |
| unidentified | 1%w |

Phenolic hydroxyl content: 0.68 eq./100 g. (calculated: 0.70).

Example II

Preparation of diglycidyl ether of DPH; method A

Crude diphenylol heptane from Example I (113.6 g; 0.772 phenolic hydroxyl equivalent) was

dissolved in a mixture of epichlorohydrin (444 g; 4.8 mol.), i-propyl alcohol (259 g) and water (64.8 g). The solution was heated to 45°C, and aqueous sodium hydroxide (160 g of 20%w solution; 0.8 mol. NaOH) was added gradually in 40 minutes with stirring. The temperature rose to 50°C. The mixture was kept at 60°C during a further 20 minutes with stirring, then the stirrer was turned off. The mixture separated immediately into two phases, and the lower phase, a weakly alkaline sodium chloride solution, was drained off. Aqueous sodium hydroxide (40 g of the 20%w solution; 0.2 mol. NaOH) was added again, and the mixture was stirred vigorously for 5 minutes at 60°C. The stirrer was turned off, the alkaline aqueous phase — the lower layer — was drained off. The organic phase was washed consecutively with water (160 ml), 2%w aqueous $NaH_2PO_4$ (160 ml) and water (160 ml). Solvent and excess epichlorohydrin were distilled off; the last traces were removed by vacuum distillation and steam vacuum distillation as in Example I.

The glycidyl ether, a pale liquid (150 g) had the properties:

| | |
|---|---|
| epoxy molar mass | 213 (calculated 203) |
| viscosity (25°C) | 2.9 Pa.s |
| saponifiable chlorine | 250 mg/kg |
| alpha-glycol | 15 mmol./kg |
| phenolic hydroxyl | 11 mmol./kg |

Example III

Preparation of diglycidyl ether of DPH; method B

In a 5 l. round bottom flask provided with stirrer, thermometer, and Dean & Stark reflux unit DPH, (568 g; 3.86 phenolic hydroxyl equivalent) was dissolved in epichlorohydrin (1850 g; 20 ml.), containing water (18.5 g) and tetramethylammonium chloride (2.19 g of a 50%w aqueous solution). The mixture was heated to 60°C, and gradual addition of sodium hydroxide (3.90 mol., as a 50%w aqueous solution) started according to the scheme:

add 0.5%w of total amount in 15 minutes; temperature rises to 102°C;
add 1%w of total amount in 15 minutes; keep temperature at 102—104°C;
add the rest in 150 minutes, and keep temperature at 102°C.

Reflux of epichlorohydrin/water starts at 102°C. The water was separated and the epichlorohydrin returned to the reactor. After addition of the NaOH the mixture was kept for 5 minutes at 102°C, the remaining epichlorohydrin was distilled off (the last traces in vacuum), the residue heated with dilute aqueous alkali (800 ml; 2.75%w NaOH) at 108°C, the mixture diluted with toluene (1200 ml), the aqueous phase separated, and the organic phase washed with a dilute phosphate solution (1800 ml; 2%w $NaH_2PO_4$), and the toluene removed in vacuum.

The glycidyl ether, a pale liquid (750 g) had the following properties.

| | |
|---|---|
| epoxy molar mass | 214 |
| viscosity (25°C) | 3.0 Pa.s |
| saponifiable chlorine | 70 mg/kg |
| alpha-glycol | 19 mmol./kg |
| phenolic hydroxyl | <10 mmol./kg |
| solubility (%w) of | |
| n-heptane | 13 |
| white spirit (b.p. 140—165°C) | 20 |
| solvent T (b.p. 184—211°C; <0.5%w | >6 |
| viscosity of the diglycidyl ether diluted with 5%w of spirit | 1 Pa.s (25°C) |

It should be noted that the commercial diglycidyl ether of 2,2-bis(4-hydroxyphenyl)propane could not be diluted with any of these aliphatic solvents.

Example IV

Cure with polyamines in solventless coating formulations

Polyether H: diglycidylether of DPH having epoxy molar mass 219.

Polyether A: diglycidylether of 2,2-bis(4-hydroxyphenyl)propane having epoxy molar mass 195 and viscosity 12.0 Pa.s (25°C).

Amine 15 : adduct of diaminodiphenylmethane and epoxides; the adduct had on average 3.8 NH-functions per molecule and was diluted with solvent and accelerator. Amount to react with 1 mol. epoxide: 115 g.

Formulations (parts by weight):

| Polyether | H | A |
|---|---|---|
| | 100 | 100 |
| red iron oxide | 15 | 15 |
| asbestine | 15 | 15 |
| microtalc | 10 | 10 |
| Amine 15 | 55 | 60 |
| Paint properties | | |
| viscosity (Pa.s, 23°C) | 7 | 28 |
| gel time* (minutes) | 47 | 32 |
| brush** | + | – |

* Tecam method according to BS 2782 method III D.
** Brush: applicability by brush.
\+ : good; – : poor.

The formulation with Polyether A is for comparative purposes only; the properties of the cured films are given between brackets in the Table below.

The formulations were sprayed onto degreased steel panels (paint films about 200 micrometres thick) and cured for 1 week at 10°C or at room temperature (20°C). Properties of the cured glossy films were:

| Cured at | | 10°C | 20°C |
|---|---|---|---|
| Hardness (Buchholz) | | 90 (100) | 100 (115) |
| Erichsen slow penetration (mm) | | 7 (0.5) | 4.5 (1) |
| Impact resistance (cm.kg) direct | | 11 (2) | 12.5 (7) |
| Resistance to aqueous: | | | |
| formic acid (5%w) | 2 weeks | u (u) | u (u) |
| $H_2SO_4$ (5%w) | 8 weeks | u (u) | u (u) |
| NaOH (5%w) | 5 weeks | u (u) | u (u) |
| Resistance to water at 70°C (8 weeks) | | u (u) | u (u) |
| Resistance to xylene at 20°C | | u (u) | u (u) |
| Salt spray (10 days) (mm from scratch) | | 2 (3) | 2 (2) |
| Humidity (7 weeks) | | + (+) | + (+) |

u = unaffected; + = resistance good

Example V

Cure with polyamine in solventless coating formulations.

The formulations with Polyethers A and AD are for comparative purposes only.

The Polyether H in this and the following Examples had epoxy molar mass 213.

Polyether A: see Example IV.

Polyether AD: a commercial liquid epoxy resin of low viscosity, recommended for coating, laminating, and civil engineering purposes; it contains Polyether A and a reactive diluent. The viscosity was 1.8 Pa.s (25°C) and the epoxy molar mass 200.

Polyether A/H is a mixture of Polyethers A and H in a 70:30 weight ratio.

Amine 20 is a liquid amine curing agent; it contains a modified cycloaliphatic polyamine and a diluent. Amount to react with 1 mol. epoxide: 115 g.

Formulations, paint properties and mechanical properties are collected in the following Table.

| | | | | |
|---|---|---|---|---|
| Formulations (part by weight) | | | | |
| Polyether | H | AD | A | A/H |
| | 70.32 | 70.32 | 70.32 | 70.32 |
| Additives | 29.68 | 29.68 | 29.68 | 29.68 |
| Amine 20 | 38.0 | 41.5 | 43.3 | 41.5 |
| Paint properties | | | | |
| viscosity (Pa.s, 23°C) | 2.7 | 3.4 | 10.8 | 8.4 |
| gel time (minutes) | 68 | 51 | 42 | 43 |
| Cured films (7 days, 23°C) | | | | |
| hardness (Buchholz) | 85 | 90 | 100 | 83 |
| gloss (%; 60°) | 95 | 95 | 98 | 95 |
| Erichsen slow penetration (mm) | 5.0 | 3.5 | 2.8 | 2.4 |
| impact resistance (direct; cm.kg) | 16 | 13.5 | 11 | 11 |
| mandrel bend, pass mm | 3 | 9 | 9 | 15 |

The additives in this Example consisted of:

| | |
|---|---|
| red iron oxide | 10.60 parts by weight |
| microtalc | 7.00 ,, ,, ,, |
| Mg silicate filler | 10.60 ,, ,, ,, |
| thixotropic agent ST | 1.40 ,, ,, ,, |
| anti-foaming agent MOS, | 0.08 ,, ,, ,, |
| | 29.68 ,, ,, ,, |

Polyether and additives were blended in a high-speed mixer, at a temperature below 60°C. Amine 20 was added just before use.

The gel time was determined as in Example IV.

All paints could be applied by brush.

Film properties were measured on 150 micrometre films (hardness and gloss: on glass plate, paint applied by doctor blade; other properties: on grit-blasted mild steel panels, paint applied by brush).

The Table demonstrates properties of the various blends and demonstrates advantages by the use of Polyether H over the use of Polyethers A and AD.

The Table demonstrates the low viscosity and the long usable pot life of the paint based on the Polyether H. It further demonstrates the good hardness of the films obtained with Polyether H, and the improved flexibility (slow penetration, impact resistance, mandrel bend), as compared with films obtained from Polyethers AD and A. Polyether A/H demonstrates that such mixtures can be used.

With respect to the chemical resistance it can be noted that all cured films were unaffected by 28 days' immersion in:

deionized water at 23°C and 70°C,
aqueous NaOH (5%w) at 23°C,
aqueous $H_2SO_4$ (5%w) at 23°C.

Upon immersion in xylene and methyl isobutyl ketone during 28 days at 23°C a slight softening occurred, but the films recovered in adhesion and strength on removal from the solvent and drying.

## Example VI

Cure with polyamine in high-solids paint formulations.

Polyether A and Amine 20 are as in Example V, Amine 15 as in Example IV. Solvent T is a commercial high-boiling aliphatic hydrocarbon solvent, boiling range 184—211°C, aromatics <0.5%w; it is used as a viscosity-cutting agent.

Formulations, paint properties, and evaluation results are collected in the following Table:

| Formulations (parts by weight) | | |
|---|---|---|
| Polyether H | 50.7 | 50.7 |
| Amine 15 | 24.8 | – |
| Amine 20 | – | 24.8 |
| Additives | 46.9 | 46.9 |
| Solvent T | 2.4 | 2.4 |
| Paint properties | | |
| viscosity (Pa.s, 23°C) | 6.3 | 2.7 |
| gel time (minutes) | 56 | 70 |
| Cured films (7 days, 23°C) | | |
| hardness (Buchholz) | 83 | 78 |
| gloss (%; 60°) | 80 | 85 |
| Erichsen slow penetration (mm) | 4.0 | 4.0 |
| impact resistance (direct; cm.kg) | 20 | 25 |
| chemical resistance (6 months' immersion) | | |
| water, 70°C | + | + |
| artificial sea water, 23°C | u | + |
| aqueous NaOH (5%w) | u | u |
| aqueous $H_2SO_4$ (5%w) | + | – |
| salt spray resistance (1000 hours) | u | u |
| humidity test (1000 hours) | u | u |

u = unaffected

\+ = resistance good (loss of gloss)

– = failed (loss of adhesion)

The additives in this Example consisted of:

| | | |
|---|---|---|
| red iron oxide | 21.0 | parts by weight |
| baryte | 6.5 | ,, ,, ,, |
| silicate filler | 18.6 | ,, ,, ,, |
| thixotropic agent ST | 0.75 | ,, ,, ,, |
| anti-foaming agent MOS | 0.05 | ,, ,, ,, |
| | 46.9 | ,, ,, ,, |

The paints were prepared as in Example V. The gel time was determined as in Example IV. Film properties were measured on 150 micrometre thick films (applied as in Example V).

The Table demonstrates that aliphatic hydrocarbon diluents can be used as a viscosity-cutting agent, that paints having good viscosity and usable pot life can be obtained, and that the films have good mechanical properties and good chemical resistance.

Example VII

Aqueous coating formulation

| | |
|---|---|
| Polyether H | 100.0 parts by weight |
| surfactant (nonylphenol ethoxylate) | 2.5 parts by weight |
| titanium dioxide pigment | 82.0 parts by weight |
| dolomite (filler) | 10.25 parts by weight |
| China clay (filler) | 5.25 parts by weight |

The components were mixed in these weight amounts, and the 200 parts by weight of paint base were mixed before use with 202 parts by weight of curing agent "Epilink" 360 in 25%w aqueous dispersion (a polyamide-type curing agent; "Epilink" is a registered trade mark).

| | |
|---|---|
| Paint properties | |
| viscosity (Pa.s, 23°C) | 41.6 |
| gel time (Tecam, minutes) | 680 |
| Film properties (cured 7 days at 23°C) | |
| thickness (micrometre) | 80 |
| gloss (%; 60°) | 75 |
| hardness (Buchholz) | 91 |
| Erichsen slow penetration (mm) | 4.9 |

Although the paint was applied here as in Example V, for determination of properties, it could also be applied for the sealing or coating of cementitious surfaces. The mixture of Polyether H and surfactant had excellent "freeze/thaw" stability: it remained clear after 3 months' temperature cycling (1 cycle = 12 hours/10°C + 12 hours/23°C).

Example VIII

A self-levelling flooring composition having a filler/binder weight ratio of about 1.5:1 was prepared from the components:

| | | |
|---|---|---|
| Formulation | | |
| | Polyether H | 100 parts by weight |
| | solvents | 9 parts by weight |
| | flow control agent | 8 parts by weight |
| | pigments — white paste $TiO_2$ | 5 parts by weight |
| | black paste FeO | 1 parts by weight |
| | curing agent Amine 35 | 35 parts by weight |
| | quartz flower | 228 parts by weight |
| Flow-out | | excellent |

Cured floorings (7 days, 23°C) according to ASTM D 790:

| | | |
|---|---|---|
| flexural strength $MN/m^2$ | | 8.0 |
| flexural modulus $MN/m^2$ | | 1120 |
| deflection % | | >5 |
| chemical resistance to | water | ++ |
| | $H_2SO_4$ (30%w) | ++ |
| | NaOH (10%w) | ++ |
| | methanol | + |

Amine 35 is a mixture of cycloaliphatic diamines containing a diluent; amount to react with 1 mol epoxide: 75 g.

In the Table ++ means very good; + means good.

Example IX

Trowelling flooring compositions were prepared from Polyether H (100 pbw), flow control agent (10 pbw), Amine 35 (35 pbw), and quartz sand filler (1—2 mm: 65%w; 0.2—0.5 mm: 35%w) in various filler/binder weight ratios. The compositions were trowelled by hand to give a thickness of 1—2 cm, and allowed to cure for 7 days at 23°C. Properties are collected in the following Table:

| Filler/binder weight ratio | 8:1 | 10:1 | 12:1 |
|---|---|---|---|
| trowelling characteristics | excellent | good | good |
| density (kg/l) | 2.2 | 2.1 | 2.1 |
| flexural strength ($MN/m^2$) | 26 | 21 | 18 |
| compressive strength ($MN/m^2$) | 76 | 64 | 58 |

When in the first formulation the Polyester H was replaced by the equivalent amount of Polyether A, the trowelling was difficult in the first formulation, and very difficult in the other formulations.

Example X

Polyether H was cured with polycarboxylic acid anhydride and mechanical and electrical properties were determined. The data are collected in the following Table:

| Compositions (pbw) | | | | | |
|---|---|---|---|---|---|
| Polyether H | | 100 | 100 | 100 | 100 |
| hexahydrophthalic anhydride | | 70 | 70 | – | – |
| benzyldimethylamine (catalyst) | | 0.5 | 0.5 | – | – |
| methyltetrahydrophthalic anhydride (+ catalyst) | | – | – | 75 | 75 |
| silica | | – | 300 | – | 300 |
| Properties (cure 3 h/80°C + 16 h/120°C) | | | | | |
| heat deflection temperature | °C | 85 | – | 92 | – |
| tensile strength | $MN/m^2$ | 66 | 68 | 54 | 66 |
| elongation | % | 4.7 | 1.3 | 4.1 | 1.2 |
| flexural strength | | | | | |
| at 5% strain | $MN/m^2$ | 90 | – | 92 | – |
| at break | $MN/m^2$ | – | 98 | – | 94 |
| compressive strength | | | | | |
| at yield | $MN/m^2$ | 95.2 | – | 93.7 | – |
| at break | $MN/m^2$ | – | 172 | – | 164 |
| volume resistivity | ohm/cm | $3.0 \times 10^{15}$ | $5.0 \times 10^{14}$ | $2.7 \times 10^{15}$ | $8.0 \times 10^{14}$ |
| surface resistivity | ohm | $4.0 \times 10^{15}$ | $2.0 \times 10^{14}$ | $2.0 \times 10^{16}$ | $2.5 \times 10^{15}$ |
| electrical strength | KV/mm | 11–12 | 11–12 | 11–12 | 11–12 |
| dielectric loss factor | | 0.004 | 0.029 | 0.003 | 0.029 |

Example XI

Preparation of glycidyl polyethers having higher epoxy molar mass, according to the general scheme:

Polyether and bisphenol were heated to 110°C, catalyst solution was added (0.2 ml), the temperature was allowed to rise to 165°C in 75 minutes, and kept at 165°C during 2½ hours. Further addition of catalyst solution and heating at 165°C (0.1 ml + 1 hour, 0.1 ml + ½ hour) were required to bring the reaction to completion. The polyether product was cooled to room temperature. The catalyst solution was a 50 weight% solution of tetramethylammonium chloride.

(a) Polyether H (3.5 epoxy equivalents) + 1,1-bis(hydroxyphenyl)heptane (1.738 phenolic hydroxyl equivalents); product: tacky solid, epoxy content 1790 meq/kg, phenolic hydroxyl <10 mmol/kg; coded Polyether HH.

(b) Polyether H (3.5 epoxy equivalents) + 2,2-bis(4-hydroxyphenyl)propane (1.71 phenolic hydroxyl equivalents); product; tacky solid, epoxy content 1859 meq/kg, phenolic hydroxyl 10 mmol/kg; coded Polyether HA.

(c) Polyether A (3.5 epoxy equivalents) + 1,1-bis(hydroxyphenyl)heptane (1.58 phenolic hydroxyl equivalents); product: tacky solid, epoxy content: 2205 mmol/kg, phenolic hydroxyl <10 mmol/kg; coded Polyether AH.

Example XII

Use of the glycidyl polyether of Example XI (c) in a coating formulation. For comparison a commercial solid glycidyl polyether of 2,2-bis(4-hydroxy)propane having epoxy molar mass 483 was used (coded Polyether AA).

Paints were prepared from the following components:

| | AH | AA |
|---|---|---|
| Polyether, 75 wt. % solution in xylene | 150 | 150 |
| xylene | 137.0 | 30 |
| n-butanol (NBA) | – | 22.5 |
| methyl ethyl ketone (MEK) | – | 22.5 |
| zinc phosphate (pigment) | 28.0 | 28.0 |
| iron oxide (pigment) | 154.5 | 154.5 |
| micro-talc (filler) | 30.5 | 30.5 |
| xylene/NBA/MEK wt. ratio 6:2:2 | – | 62.0 |

In the case of Polyether AH xylene could be used as sole solvent; for Polyether AA the presence of NBA and MEK was required.

The curing agent (a polyaminoamide derived from dimerized fatty acids in a 75 wt.% solution in xylene) was added immediately before use of the paint, in a weight ratio resin/curing agent of 100:40 (both calculated on solids).

The paints were then sprayed onto degreased, shot-blasted, cold-rolled mild steel panels (film thickness about 25 micrometres), and allowed to cure at 23°C during 7 days. Results of mechanical and chemical evaluation are given in the Table below.

| Paint based on Polyether | AH | AA |
|---|---|---|
| Mechanical | | |
| Hardness (Buchholz) | 95 | 100 |
| (König, sec.) | 147 | 111 |
| Erichsen, slow penetration (mm) | pass 8 | pass 8 |
| Gardner impact resistance (mm) | | |
| direct | 30 | 30 |
| reverse | 20 | 20 |
| Mandrel bend (conical) | pass | pass |
| Crosshatch adhesion | Gt 0 | Gt 0 |
| Chemical resistance to: | | |
| distilled water, 70°C, 8 weeks | 10 | 10 |
| 5 wt. % caustic, 20°C, 8 weeks | 4 VF | 8 MD |
| 5 wt. % sulphuric acid, 20°C, 8 weeks | 8 VF | 2 VF |
| xylene, 20°C, 8 weeks | 10 | 10 |
| MIBK, 20°C, 8 weeks | 10 | 10 |
| salt spray 240 hours | 2 mm + 10 | 1 mm + 8 F |
| 500 hours | 2 mm + 10 | 5 mm + 8 MD |
| 750 hours | 2 mm + 6 F | 5 mm + 6 MD |
| 1500 hours | 2 mm + 4 F | 5 mm + 4 MD |
| humidity 1500 hours | 10 | 10 |

Assessments for chemical resistance are given according to the ASTM blister rating.

For salt spray resistance the panels were scratched, and rust from scratch + blister rating were recorded.

The Table shows that the Polyether AH performs similar to the commercial Polyether AA in mechanical properties, and performs even better in chemical resistance, in particular salt spray resistance.

Example XIII

Anionic resin and use in an anodic electrodeposition paint.

(a) Polyether HH of Example XI (559 g; 1 epoxy equivalent) was dissolved in toluene (65 g) at 140°C under reflux. Adipic acid (36.5 g; 0.25 mol), dimethylolpropionic acid (67 g; 0.5 mol), and benzyldimethylamine (1.6 g; catalyst) were added, and the mixture was allowed to react at 140—145°C during 5 hours with stirring; the acid content was then 0.03 meq/g. Then a solution of trimellitic anhydride (48 g; 0.25 mol)in acetone (100 g) was added gradually in $\frac{1}{2}$ h; the volatiles (acetone and toluene) were distilled off, and the temperature was kept at 135—140°C for a further $\frac{1}{2}$ hour. Cooling to room temperature provided a clear solid resin having an acid content of 0.66 meq/g and about 2%w volatiles.

(b) The resin of (a) (116.3 g, 114 g solids) was dissolved in ethylene glycol monobutylether (49 g) at 75—80°C; the solution was cooled to room temperature, blended with hexamethoxymethyl melamine (6.0 g; cross-linking agent) and triethylamine (6.2 g; for neutralization), and diluted with demineralized water (267 g) to provide an aqueous solution of 27 wt.% solids. 200 g of this solution were pigmented with titanium dioxide (27 g), clay DSP—100 (1.5 g) and carbon black (1.5 g) in a sand mill during 45 minutes. The pigment paste was thinned with the remaining

binder solution (244 g) and demineralized water (526 g); the resulting grey paint had solids content of 15%w, pH 7.2, and specific conductivity 950 micro S/m (25°C).

(c) The paint of part (b) was electrodeposited anodically onto degreased cold-rolled steel panels (thickness 0.7 mm) at 25°C, 150—175 V, during 2 minutes. The coated panels were rinsed with water, blown dry, and stoved at 180°C during 30 minutes. Properties are collected in the following Table.

| | |
|---|---|
| Appearance before stoving | smooth, semi-gloss |
| Appearance after stoving | smooth, glossy |
| Film thickness (micrometre) | 23–25 |
| MEK rubs | >50 |
| Impact resistance (cm.kg) | >90 |
| Salt spray resistance (10 days) | |
| mm rust creep | 3–5 |

When the Polyether HH in this formulation was replaced by a similar polyether drived from 2,2-bis(4-hydroxyphenyl)propane, the appearance before and after stoving was rough.

Example XIV

Cationic resin and use in cathodic electrodeposition paint.

(a) Polyether HH of Example XI (838.5 g; 1.5 epoxy equivalents) was dissolved in ethylene glycol monobutylether (354 g) at 120°C and reacted with a mixture of diethanolamine (52.5 g; 0.5 mol), ethanolamine (26.7 g; 0.438 mol), sulphanilic acid (10.8 g; 0.062 g) in water (12.5 g) at 120°C during 3 hours. This product was then reacted at 120°C for $1\frac{1}{2}$ hours with a commercial glycidyl ester of saturated aliphatic monocarboxylic acids in which the carboxyl group is attached to a tertiary or quaternary carbon atom and which carboxylic acids have on average 10 carbon atoms per molecule (46.8 g; 0.187 epoxy equivalent). The clear viscous resin solution had solids content 72.7%w, amino-N content 0.75 meq/g, acid content 0.03 meq/g, and residual epoxy content 0.02 meq/g.

(b) The resin solution of (a) was blended with hexamethoxymethyl melamine in a solids weight ratio of 95:5, with acetic acid for neutralilization of 40% of the amino groups, and then diluted with demineralized water to solids content 10%w, pH = 4.7.

This solution was cathodically electrodeposited onto degreased cold-rolled steel panels (25°C; 175 V; 2 minutes), the panels were rinsed with water and dried, and stoved at 180°C during 30 minutes. Properties are collected in the following Table.

| | |
|---|---|
| Appearance before stoving | smooth, silky |
| Appearance after stoving | smooth, glossy |
| Film thickness (micrometre) | 18–20 |
| MEK rubs | >50 |
| Impact resistance (cm.kg) | >90 |
| Salt spray resistance (20 days) | |
| mm rust creep | < 3 |

Example XV

Cationic resin, cure with a non-acidic polyester and a metal compound, cathodic electrodeposition.

(a) Polyether HA of Example XI (403.5 g; 0.75 epoxy equivalent) was dissolved in ethylene glycol monobutylether (94 g) at 60°C, and reacted with a mixture of diethanolamine (26.2 g; 0.25 mol), ethanolamine (7.6 g; 0.125 mol) in ethylene glycol monobutylether (54 g) at 75—80°C during $\frac{1}{2}$

hour (epoxy content 0.43 meq/g). A solution of 3-dimethylaminopropylamine (12.7 g; 0.125 mol) in ethylene glycol monobutylether (45 g) was added, and the mixture was reacted 1 hour at 80°C and 1 hour at 120°C. The resulting solution (70%w solids) had amino content 0.99 meq/g; epoxy content zero.

(b) A polyester cross-linking agent was prepared by reacting trimellitic anhydride (192 g; 1 mol) and glycidyl ester of monocarboxylic acid as used in Example XIV (a) (500 g; 2 mol) with stirring. The exothermic reaction started at 100°C, the temperature was allowed to rise to 190°C. After cooling to 140°C, benzyl dimethylamine (2 g; catalyst) was added, and the mixture was kept at 140°C during 3 hours. The viscous, clear product had a residual acid content of 0.053 meq/g and a molecular weight (GPC) of about 3000.

(c) A cathodic electrodeposition paint was prepared by mixing resin solution of (a) (125.1 g), polyester of (b) (32.4 g), lead 2-ethyl hexoate (2.4 g of a commercial product containing 33%w Pb), acetic acid (3.7 g), ethylene glycol monobutyl ether (13.9 g), and water (251 g) to form an aqueous binder of 28%w solids content, 200 g of this binder were pigmented with red iron oxide (28 g) and clay DSP—100 (2 g) in a sand mill during 45 minutes. This pigment paste was thinned with the remaining aqueous binder and water (542 g); the resulting aqueous paint (15%w solids; pH = 5.8; specific conductivity 1600 micro S at 25°C) was electrodeposited cathodically onto degreased, cold-rolled steel panels of 0.7 mm thickness at 25°C during 2 minutes at 175 V. The panels were rinsed with water, dried and stoved at 180°C during 30 minutes. Coating properties were:

| | |
|---|---|
| Rupture voltage (v) | 350 |
| Appearance before stoving | smooth, silky |
| Appearance after stoving | smooth, semi-gloss |
| Film thickness (micrometre) | 13–15 |
| MEK rubs | >50 |
| Impact resistance (cm.kg) | >90 |
| Salt spray resistance (20 days) | |
| mm rust creep | 3–5 |

## Claims

1. Polyglycidyl ethers of diphenylol alkanes having the general formula:

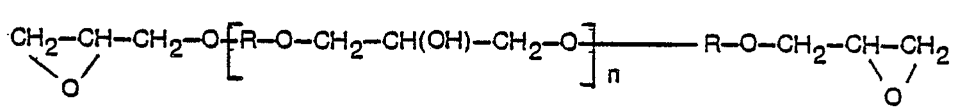

(II)

wherein n is a number of from 0 to 12 and R is the hydrocarbon residue of a diphenylol alkane, characterized in that from 1 to (n + 1) groups R in the above molecule have the formula:

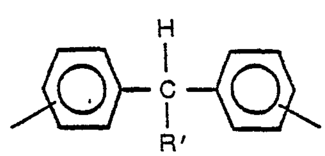

wherein R′ is a n-hexyl group and wherein the weight ratio of para, para′-isomer to ortho, para′-isomer is from 90:10 to 40:60.

2. Process for the preparation of polyglycidyl ethers of diphenylol alkanes as claimed in claim 1,

wherein a diphenylol alkane is reacted with epichlorohydrin or with a polyepoxide, characterized in that the diphenylol alkane has the general formula:

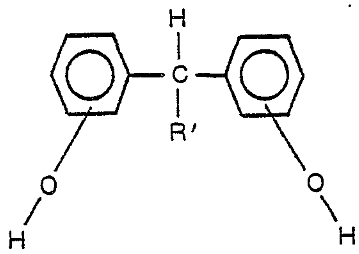

wherein R′ is a n-hexyl group and the weight ratio of para,para′-isomer to ortho,para′-isomer is from 90:10 to 40:60.

3. A polyglycidylether as claimed in claim 1, characterized in that n is a number of from 0 to 4.

4. A polyglycidyl ether as claimed in claim 1 or 3, characterized in that in the formula of R the weight ratio of para, para′-isomer to ortho-para′-isomer is from 80:20 to 40:60.

5. Process for curing a polyglycidyl ether as claimed in any of claims 1, 3 or 4, comprising mixing and reacting the polyglycidyl ether with a curing amount of an epoxy resin curing agent.

6. Process as claimed in claim 5, characterized in that the curing agent is an amino compound, a polycarboxylic acid, or a polycarboxylic acid anhydride.

7. Use of a polyglycidyl ether as claimed in any of claims 1, 3 or 4 for the preparation of anionic or cationic aqueous thermosetting paint binders.

**Patentansprüche**

1. Polyglycidylether von Diphenylolalkanen der allmeinen Formel

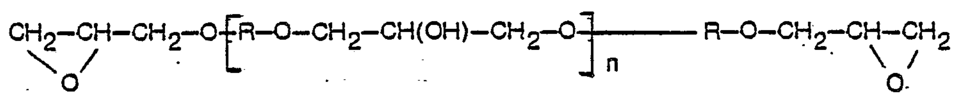

(II)

in der n eine Zahl von 0 bis 12 bedeutet und R der Kohlenwasserstoffrest eines Diphenylolalkans ist, dadurch gekennzeichnet, daß 1 bis (n + 1) Gruppen R in dem obigen Molekül die Formel

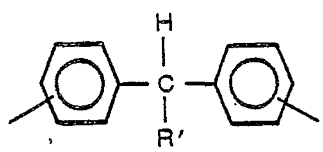

haben, in der R′ eine n-Hexylgruppe ist und wobei das Gewichtsverhältnis von p, p′-Isomer zu o, p′-Isomer 90:10 bis 40:60 beträgt.

2. Verfahren zur Herstellung von Polyglycidylethern von Diphenylolalkanen nach Anspruch 1 durch Umsetzen eines Diphenylolalkans mit Epichlorhydrin oder mit einem Polyepoxid, dadurch gekennzeichnet, daß das Diphenylolalkan die allgemeine Formel

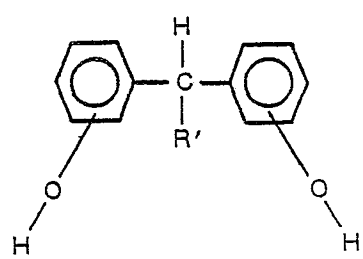

aufweist in der R′ eine n-Hexylgruppe ist und das Gewichtsverhältnis von p, p′-Isomer zu o, p′-Isomer 90:10 bis 40:60 beträgt.

3. Polyglycidylether nach Anspruch 1, dadurch gekennzeichnet, daß n eine Zahl von 0 bis 4 ist.

4. Polyglycidylether nach Anspruch 1 oder 3, dadurch gekennzeichnet, daß in der Formel von R das Gewichtsverhältnis von p, p'-Isomer zu o, p'-Isomer 80:20 bis 40:60 beträgt.

5. Verfahren zum Härten eines Polyglycidylethers nach einem der Ansprüche 1, 3 oder 4, bei dem der Polyglycidylether mit einer härtenden Menge eines Epoxyharz- Härtungsmittels vermischt und umgesetzt wird.

6. Verfahren nach Anspruch 5, dadurch gekennzeichnet, daß das Härtungsmittel eine Aminoverbindung, eine Polycarbonsäure oder eine Polycarbonsäureanhydrid ist.

7. Verwendung eines Polyglycidylethers nach einem der Ansprüche 1, 3 oder 4 zur Herstellung von anionischen oder kationischen wässrigen Bindemitteln für wärmehärtende Anstrichmittel.

**Revendications**

1. Ethers polyglycidiques de diphénylolalcanes ayant la formule générale:

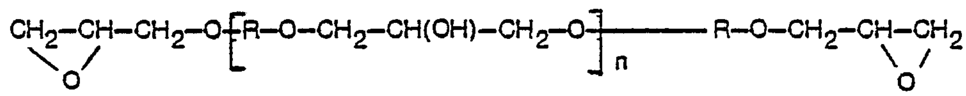

(II)

dans laquelle n est un nombre de 0 à 12 et R est le résidu d'hydrocarbure d'un diphénylolalcane, caractérisés en ce que 1 à (n +1) groupes R dans la molécule ci-dessus ont la formule:

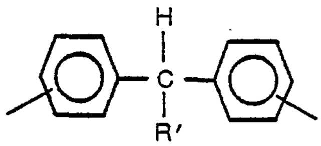

(III)

dans laquelle R' est un groupe n-hexyle et le rapport en poids de l'isomère para,para' à l'isomère ortho,para' est compris entre 90:10 et 40:60.

2. Procédé pour la préparation d'éthers polyglycidiques de diphénylolalcanes tels que revendiqués dans la revendication 1, dans lequel un diphénylolalcane est mis à réagir avec l'épichlorhydrine ou avec un polyépoxyde, caractérisé en ce que le diphénylolalcane a la formule générale:

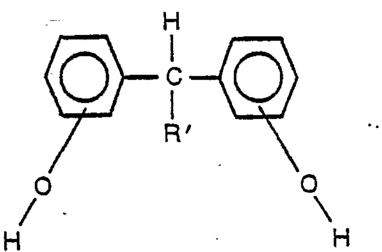

dans laquelle R' est un groupe n-hexyle et le rapport en poids de l'isomère para,para' à l'isomère ortho,para' est compris entre 90:10 et 40:60.

3. Un éther polyglycidique selon la revendication 1, caractérisé en ce que n est un nombre entier de 0 à 4.

4. Un éther polyglycidique selon la revendication 1 ou 3, caractérisé en ce que dans la formule de R le rapport en poids de l'isomère para,para' à l'isomère ortho,para' est compris entre 80:20 et 40:60.

5. Procédé pour le durcissement d'un éther polyglycidique selon l'une quelconque des revendications 1, 3 ou 4, selon lequel on mélange et on fait réagir l'éther polyglycidique avec une quantité durcissante d'un durcisseur pour résines époxy.

6. Procédé selon la revendication 5, caractérisé en ce que le durcisseur est un composé aminé, un acide polycarboxylique ou un anhydride d'acide polycarboxylique.

7. Utilisation d'un éther polyglycidique selon l'une quelconque des revendications 1, 3 ou 4 pour la préparation de liants aqueux anioniques ou cationiques pour peintures thermodurcissables.